# EUROPEAN PATENT SPECIFICATION

(11) **EP 1 248 797 B1**
(45) Date of publication and mention of the grant of the patent: **04.03.2009**
(21) Application number: 01942636.0
(22) Date of filing: 18.01.2001
(51) Int. Cl.: C07K 1/22, C07K 14/81

(54) **PROCESS FOR THE SEPARATION OF ANTITHROMBIN-III ISOFORMS**
VERFAHREN ZUR TRENNUNG VON ANTITHROMBIN-III ISOFORMEN
PROCEDE POUR LA SEPARATION D'ISOFORMES D'ANTITHROMBINE-III

(30) Priority: 21.01.2000 SE 0000177
(43) Date of publication of application: 16.10.2002
(73) Proprietor: Octapharma AG, 8853 Lachen (CH)
(72) Inventor: KARLSSON, Göran, S-178 40 Ekerö (SE)
(74) Representative: Meyers, Hans-Wilhelm
(86) International application number: PCT/SE2001/000085
(87) International publication number: WO 2001/053329

(56) References cited:
- WO-A1-01/53328
- G. KREYSA ET AL.: 'Bioprocess engineering monitoring and controlling applied genetics and safety low molecular weight metabolites environmental biotechnology' ANNUAL MEETING OF BIOTECHNOLOGISTS vol. 5, no. PART B, 1992, pages 679 - 682, XP002941310
- SIW FREBELIUS ET AL.: 'Thrombin inhibition by antithrombin III on the subendothelium is explained by the isoform AT beta' ARTERIOSCLER. THROMB. VASC. BIOL. vol. 16, no. 10, October 1996, pages 1292 - 1297, XP002941311
- MASAHIRO NIWA ET AL: 'HISTIDINE-RICH GLYCOPROTEIN INHIBITS THE ANTITHROMBIN ACTIVITY OF HEPARIN COFACTOR II IN THE PRESENCE OF HEPARIN OR DERMATAN SULFATE' THROMBOSIS RESEARCH vol. 37, 1985, pages 237 - 240

## Description

### TECHNICAL FIELD

The present invention relates to processes for the preparation of a solution comprising a substantially pure isoform of AT-III, said process comprising separating the isoform AT-IIIα from AT-IIIβ on a calcium hydroxyphosphate-based adsorbent.

### BACKGROUND ART

Antithrombin III (AT-III) is a plasma glycoprotein that inhibits serine proteases in the coagulation cascade and thus plays a major role in the regulation of blood clotting. Antithrombin III is an inhibitor of Factors IXa, Xa, XI, XIIa, and thrombin. Thus, AT-III regulates clot formation in different stages of the coagulation cascade. A small decrease of the AT-III content in the blood is associated with increased risk of thromboembolism. AT-III concentrates are used in the prophylaxis and treatment of thromboembolic disorders in patients with acquired or hereditary antithrombin deficiency. In addition, it has been reported that AT-III is involved in many other biological responses, for example angiogenesis and inflammatory responses. The function of AT-III in these mechanisms is not yet fully understood.

Purification of AT-III with affinity chromatography, using heparin as the solid phase bound ligand, is known in the art. Miller-Andersson et al. (Thrombosis Research 5, 439-452, 1974) discloses the use of heparin-Sepharose to purify human AT-III. The entire procedure, which included ion exchange and gel filtration chromatography, provided a 34% yield.

In human plasma, antithrombin III exists as at least two molecular entities, which are homologous according to amino acid composition, but differ in carbohydrate content and in their heparin-binding behavior. An antithrombin variant, designated as AT-IIIβ, was isolated from human plasma independently from the predominant antithrombin species (designated as AT-IIIα), by virtue of its tight binding to a heparin-Sepharose matrix at high ionic strengths (Peterson, C.B. & Blackburn, M.N. (1985) J. Biol. Chem. 260, 610-615).

The determined molecular weights were 59,800 and 56,900 for human AT-IIIα and AT-IIIβ, respectively. The difference in molecular weights of the two antithrombins was attributed to a reduction of approximately 25-30% in the sialic acid, neutral sugar, and amino sugar content of AT-IIIβ when compared to the carbohydrate content of the AT-IIIα subspecies (Peterson & Blackburn, *supra*). It has been shown that AT-IIIβ lacks one of the four oligosaccharide side-chains, namely the side-chain at asparagine 135 (Brennan, S.O. et al. (1987) FEBS Letters 219, 431-436). The AT-IIIα form is more negatively charged than AT-IIIβ; it has been demonstrated that AT-IIIα and AT-IIIβ have pl:s of 4.9 and 5.1, respectively (Frebelius, S. et al. (1996) Arteriosclerosis, Thrombosis, and Vascular Biology 16:1292-1297).

It is desirable to obtain pure AT-IIIβ, as this form has specific effects on the coagulation in the vessel wall. It has been shown that AT-IIIβ can prevent restenosis of the rabbit aorta after balloon injury (Swedenborg (1998) Blood Coagulation and Fibrinolysis 9 (suppl. 3):S7-S10). AT-IIIβ may therefore be considered as a potential drug for humans in prophylaxis of restenosis when performing balloon dilatation of the aorta.

Histidine-rich glycoprotein (HRGP) is a single-chained plasma protein originally isolated in 1972. The exact physiological function of HRGP is still unknown. Due to interaction with heparin, fibrinogen and fibrin, plasminogen and activated platelets, HRGP is considered to be a modulator of coagulation and fibrinolysis (Koide, T. In: Fibrinolysis: Current Prospects. Gaffney, PJ (Ed.), John Libbey & Co., London 1988, p.55-63). The polypeptide chain consists of 507 amino acid residues and contains regions that share homology with other plasma proteins, e.g. antithrombin-III (Koide, T. et al. (1986) Biochemistry 25, 2220-2225).

As indicated above, the complete involvement of the two AT-III isoforms and HRGP in the body is not yet fully understood. Consequently, it is desirable to provide efficient purification methods for producing the proteins in pure form, which will facilitate studies *in vivo* and *in vitro.*

The use of hydroxyapatite, by means of displacement chromatography, has been disclosed in the purification of antithrombin-III (Freitag & Breier (1995) J. Chromatography A, 691, 101-112 as well as Kreysa et al., Annual Meeting of Biotechnologists (1992) vol. 5, p.679-682). However, the purification of separate isoforms of AT-III has not previously been achieved by hydroxyapatite chromatography.

### BRIEF DESCRIPTION OF THE DRAWING

### Fig. 1

Separation of AT-IIIα and AT-IIIβ by hydroxyapatite chromatography

### DISCLOSURE OF THE INVENTION

It has surprisingly been shown that the antithrombin III isoforms, AT-IIIα and AT-IIIβ, can be conveniently separated and purified using a calcium hydroxyphosphate-based adsorbent.

Consequently, this invention provides in a first aspect a process for the preparation of a solution comprising a substantially pure isoform of AT-III, comprising separating the isoform AT-IIIα from AT-IIIβ on a calcium hydroxyphosphate-based adsorbent. The said process preferably comprising the steps:
(i) preparing a solution mainly comprising AT-III;
(ii) contacting the said solution with the calcium hydroxyphosphate-based adsorbent, preferably hydroxyapatite;
(iii) by adsorption chromatography, preferably column chromatography, eluting and collecting the protein fraction comprising the substantially pure isoform of AT-III.

The said solution mainly comprising AT-III can conveniently be prepared according methods known in the art. A suitable method could e.g. include the steps:
(i) preparing a Cohn Fraction I supernatant from human plasma by known methods (see e.g. Cohn et al. (1946) J. Am. Chem. Soc. 68, 459-475);
(ii) contacting the said Cohn Fraction I supernatant with an affinity gel capable of binding AT-III (see e.g. Koide, T. et al. (1985) J. Biochem. 98, 1191-1200); and
(iii) eluting and collecting the protein fraction binding to the said affinity matrix.

The said affinity gel preferably comprises heparin as the affinity ligand. Suitable affinity gels include heparin-Sepharose® (Amersham Pharmacia); HeparinHyperD (Biosepra), Fractogel TSK AF-Heparin 650 (Merck), Heparin-Agarose (Sigma), TSKgel Heparin (Tosohaas), Heparin-Agarose 6XL (ACL).

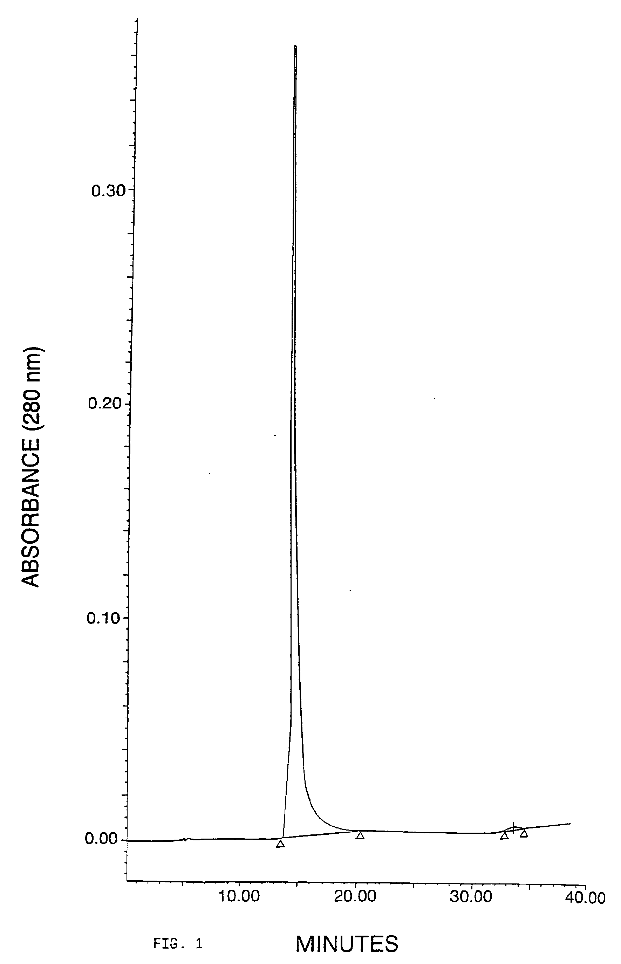

As shown in Example 1 below, AT-IIIα is normally eluted from the calcium hydroxyphosphate-based adsorbent with a buffer having a phosphate concentration from about 50 to about 150 mM. AT-IIIβ is normally eluted from the calcium hydroxyphosphate-based adsorbent with a buffer having a phosphate concentration above 150 mM to about 400 mM, preferably from about 200 to about 300 mM. As shown in Example 1 and Fig. 1, AT-IIIα and AT-IIIβ can be collected in fractions eluting from the chromatography column at concentration of about 110 mM and about 250 mM, respectively. The obtained isoform of AT-III is substantially free from histidine-rich glycoprotein (HRGP).

The adsorbent used in this invention could also account for the preparation of a solution comprising a substantially pure histidine-rich glycoprotein (HRGP), e.g. when the starting material, in addition to AT-III, also comprises some amount of HRGP, which will normally be the case when the above-described method for preparing AT-III is used (cf. Koide, T. et al. (1985) J. Biochem. 98, 1191-1200). The skilled person will be able to determine the conditions suitable for eluting HRGP from the chromatography column. Under the conditions used in Example 1 below, HRGP will normally be eluted from the column at a phosphate concentration of from about 300 to about 400 mM, such as about 340 mM.

The process according to the invention is normally carried out at a pH from about 6.0 to about 7.5, preferably from about 6.5 to about 7.2, such as about pH 6.8.

The antithrombin preparations produced according to the present invention are suitable as pharmaceutically effective ingredients in pharmaceutical compositions and combinations. The pharmaceutical compositions may optionally comprise additional active ingredients like anti-coagulants such as hirudin or heparin ,or thrombolytic agents such as plasminogen activator or hementin. The antithrombin preparations produced according to the invention may form pharmaceutically acceptable salts with any non-toxic, organic or inorganic acid.

The antithrombin preparations produced according to the invention may be administered as unit doses containing conventional non-toxic pharmaceutically acceptable carriers, diluents, adjuvants and vehicles which are typical for parenteral administration. As used herein, the term "pharmaceutically acceptable carrier" means an inert, non toxic solid or liquid filler, diluent or encapsulating material, not reacting adversely with the active compound or with the patient. Suitable, preferably liquid carriers are well known in the art such as sterile water, saline, aqueous dextrose, sugar solutions, ethanol, glycols and oils, including those of petroleum, animal, vegetable, or synthetic origin, for example, peanut oil, soybean oil and mineral oil.

The term "parenteral" includes herein subcutaneous, intravenous, intra-articular and intratracheal injection and infusion techniques. Also other administrations such as oral administration and topical application are suitable. Parenteral compositions and combinations are most preferably administered intravenously either in a bolus form or as a constant fusion according to known procedures. Tablets and capsules for oral administration contain conventional excipients such as binding agents, fillers, diluents, tableting agents, lubricants, disintegrants, and wetting agents. The tablets may be coated according to methods well known in the art.

Oral liquid preparations may be in the form of aqueous or oily suspensions, solutions, emulsions, syrups or elixirs, or may be presented as a dry product for reconstitution with water or another suitable vehicle before use. Such liquid preparations may contain conventional additives like suspending agents, emulsifying agents, non-aqueous vehicles and preservatives. Topical applications may be in the form of aqueous or oily suspensions, solutions, emulsions, jellies or preferably emulsion ointments.

Unit doses according to the invention may contain daily required amounts of the protein according to the invention, or sub-multiples thereof to make up the desired dose. The optimum therapeutically acceptable dosage and dose rate for a given patient (mammals, including humans) depends on a variety of factors, such as the activity of the specific active material employed, the age, body weight, general health, sex, diet, time and route of administration, rate of clearance. The object of the treatment, i.e., therapy or prophylaxis and the nature of the thrombotic disease to be treated, antiplatelet or anticoagulant activity.

### EXPERIMENTAL METHODS

Biological activity (IU/ml) of AT-III was determined as heparin cofactor activity by monitoring the cleavage of the chromogenic substrate H-D-Phe-Pip-Arg-pNA · 2 HC1 (Chromogenix, Sweden) by thrombin in presence of heparin and AT-III. See Frantzen Handeland et al. (Scand. J. Haematol. 31, 427-436, 1983) and van Voorhuizen et al. (Thromb. Haemostas. 52(3), 350-353, 1984).

Total protein concentration was determined by absorption measurements at 280 nm (A₂₈₀). Concentration (mg/ml) for AT-III solutions was calculated using the coefficient of 6.4 IU/mg. Specific activity (SA) of AT-III was defined as the ratio between heparin cofactor activity calculated as IU/ml and A₂₈₀.

### EXAMPLES

### EXAMPLE 1: Separation of AT-IIIα and AT-IIIβ by hydroxyapatite chromatography

A sample of human antithrombin having a purity of >95%, prepared according to methods known in the art (cf. Miller-Andersson et al. (1974) Thrombosis Research 5, 439-452) was used to analyze the alpha and beta forms of antithrombin.

The antithrombin sample was chromatographed using an HPLC system, equipped with a hydroxyapatite (75 x 7.5 mm ID, 5 µm, pore size 1000 Å) analytical column that was equilibrated in 10 mM sodium phosphate, 0.01 mM calcium chloride, pH 6.8. The injection volume of antithrombin (6.8 mg/ml) was 25 µl, and the flow rate was 0.5 ml/min at room temperature. Elution was carried out by mean of a segmented gradient; first an isocratic wash period (0 to 5 min) in equilibration buffer, then a short gradient was run (5 to 8 min) to 110 mM sodium phosphate, then a 17 min isocratic run, followed by a linear gradient (25 to 35 min) up to 500 mM sodium phosphate. Detection was carried out by measuring UV absorbance at 280 nm. AT-IIIα and AT-IIIβ eluted at the retention times 16 and 35 minutes, respectively (Fig. 1). Purified AT-IIIα and AT-IIIβ, obtained according to known methods (cf. Peterson, C.B. & Blackburn, M.N. (1985) J. Biol. Chem. 260, 610-615), were used as control samples and gave the same retention times as above when they were analyzed in the same way. No other peaks than AT-IIIα and AT-IIIβ appeared in the chromatogram. The peaks were separated with good resolution and the results showed that the AT-III sample contained about 0.7% of AT-IIIβ, based on the integrated area. Purified histidine-rich glycoprotein gave a longer retention time than AT-IIIβ when analyzed in the same system.

### EXAMPLE 2: Purification of AT-IIIα and AT-IIIβ by hydroxyapatite chromatography

An AT-III sample, 450 mg of the same quality as described in Example 1, in 30 ml 10 mM sodium phosphate, pH 6.8, is suitable to be chromatographed using a chromatographic system, equipped with a hydroxyapatite, 15 µm particle size, preparative column (35 x 2.5 cm ID) equilibrated in 10 mM sodium phosphate, 0.01 mM calcium chloride, pH 6.8. The flow rate of 3 ml/min at a temperature of +4°C to +8°C is appropriate for the purification. Elution by means of a segmented gradient: first a 45 min isocratic run in equilibration buffer during sample loading and washing, then a linear gradient (0.5 h) to 110 mM sodium phosphate, followed by an isocratic run (2.6 h), and finally a linear gradient (1.5 h) up to 500 mM sodium phosphate. Detection by measuring UV absorbance at 280 nm. AT-IIIα and AT-IIIβ elute after approximately 2 and 5 h, respectively.

Instead of hydroxyapatite having a particle size of 15 µm, other types of hydroxyapapatite could conveniently be used for purification in a larger scale, e.g. Macro-Prep® ceramic hydroxyapatite (Bio-Rad; particle size 80 µm).

## Claims

1. A process for the preparation of a solution comprising a pure isoform of AT-III, comprising separating the isoform AT-IIIα from AT-IIIβ on a calcium hydroxyphosphate-based adsorbent.

2. The process according to claim 1, comprising the steps
(i) preparing a solution comprising AT-III;
(ii) contacting the said solution with the calcium hydroxyphosphate-based adsorbent;
(iii) eluting and collecting the protein fraction comprising the pure isoform of AT-III.

3. The process according to claim 1 or 2 wherein the separation of AT-IIIα and AT-IIIβ is carried out by column chromatography.

4. The process according to claim 1 for the preparation of pure AT-IIIα.

5. The process according to claim 4 wherein AT-IIIα is eluted from the calcium hydroxyphosphate-based adsorbent with a buffer having a phosphate concentration of from 50 mM to 150 mM.

6. The process according to claim 1 for the preparation of pure AT-IIIβ.

7. The process according to claim 6 wherein AT-IIIβ is eluted from the calcium hydroxyphosphate-based adsorbent with a buffer having a phosphate concentration of from 150 mM to 400 mM.

8. The process according to claim 1 wherein the said calcium hydroxyphosphate-based adsorbent is hydroxyapatite.

9. The process according to claim 1 wherein separation of AT-IIIα and AT-IIIβ is carried out at a pH of from 6.0 to 7.5.

10. The process according to claim 2 wherein the said solution comprising AT-III is prepared by a process comprising the steps
(i) preparing a Cohn Fraction I supernatant from human plasma;
(ii) contacting the said Cohn Fraction I supernatant with an affinity gel capable of binding AT-III; and
(iii) eluting and collecting the protein fraction binding to the said affinity matrix.

11. The process according to claim 10 wherein the said affinity gel comprises heparin as the affinity ligand.

12. The process according to claim 1 wherein the obtained isoform of AT-III is free from histidine-rich glycoprotein (HRGP).

## Patentansprüche

1. Verfahren zur Herstellung einer Lösung, die eine reine Isoform von AT-III umfasst, umfassend das Trennen der Isoform AT-IIIα von AT-IIIβ auf einem Adsorbens auf Calciumhydroxyphosphat-Basis.

2. Verfahren gemäß Anspruch 1, das die folgenden Schritte umfasst:
(i) Herstellen einer Lösung, die AT-III umfasst;
(ii) In-Kontakt-Bringen der Lösung mit dem Adsorbens auf Calciumhydroxyphosphat-Basis;
(iii) Eluieren und Auffangen der Proteinfraktion , die die reine Isoform von AT-III umfasst.

3. Verfahren gemäß Anspruch 1 oder 2, wobei die Trennung von AT-IIIα und AT-IIIβ durch Säulenchromatographie durchgeführt wird.

4. Verfahren gemäß Anspruch 1 für die Herstellung von reinem AT-IIIα.

5. Verfahren gemäß Anspruch 4, wobei AT-IIIα mit einem Puffer, der eine Phosphatkonzentration von 50 mM bis 150 mM aufweist, aus dem Adsorbens auf Calciumhydroxyphosphat-Basis eluiert wird.

6. Verfahren gemäß Anspruch 1 für die Herstellung von reinem AT-TIIβ.

7. Verfahren gemäß Anspruch 6, wobei AT-IIIβ mit einem Puffer, der eine Phosphatkonzentration von 150 mM bis 400 mM aufweist, aus dem Adsorbens auf Calciumhydroxyphosphat-Basis eluiert wird.

8. Verfahren gemäß Anspruch 1, wobei es sich bei dem Adsorbens auf Calciumhydroxyphosphat-Basis um Hydroxyapatit handelt.

9. Verfahren gemäß Anspruch 1, wobei die Trennung von AT-IIIα und AT-IIIβ bei einem pH-Wert von 6,0 bis 7,5 durchgeführt wird.

10. Verfahren gemäß Anspruch 2, wobei die Lösung, die AT-III umfasst, durch ein Verfahren hergestellt wird, das die folgenden Schritte umfasst:
(i) Herstellung eines Überstands der Cohn-Fraktion I aus humanem Plasma;
(ii) In-Kontakt-Bringen des Überstands der Cohn-Fraktion I mit einem Affinitätsgel, das AT-III binden kann; und
(iii) Eluieren und Auffangen der Proteinfraktion, die an die Affinitätsmatrix bindet.

11. Verfahren gemäß Anspruch 10, wobei das Affinitätsgel Heparin als Affinitätsligand umfasst.

12. Verfahren gemäß Anspruch 1, wobei die erhaltene Isoform von AT-III frei von histidinreichem Glycoprotein (HRGP) ist.

## Revendications

1. Procédé de préparation d'une solution comprenant une isoforme pure de AT-III, comprenant l'étape consistant à séparer l'isoforme AT-IIIα de AT-IIIβ sur un adsorbant à base d'hydroxyphosphate de calcium.

2. Procédé selon la revendication 1, comprenant les étapes consistant à :
(i) préparer une solution comprenant AT-III;
(ii) mettre en contact ladite solution avec l'adsorbant à base d'hydroxyphosphate de calcium ;
(iii) éluer et recueillir la fraction protéinique comprenant l'isoforme pure de AT-III.

3. Procédé selon la revendication 1 ou 2, dans lequel la séparation de AT-IIIα et de AT-IIIβ est réalisée par chromatographie sur colonne.

4. Procédé selon la revendication 1, pour la préparation de AT-IIIα pur.

5. Procédé selon la revendication 4, dans lequel AT-IIIα est élué de l'adsorbant à base d'hydroxyphosphate de calcium avec un tampon ayant une concentration en phosphate allant de 50 mM à 150 mM.

6. Procédé selon la revendication 1, pour la préparation de AT-IIIβ pur.

7. Procédé selon la revendication 6, dans lequel AT-IIIβ est élué de l'adsorbant à base d'hydroxyphosphate de calcium avec un tampon ayant une concentration en phosphate allant de 150 mM à 400 mM.

8. Procédé selon la revendication 1, dans lequel ledit adsorbant à base d'hydroxyphosphate de calcium est l'hydroxyapatite.

9. Procédé selon la revendication 1, dans lequel la séparation de AT-IIIα et de AT-IIIβ est réalisée à un pH allant de 6,0 à 7,5.

10. Procédé selon la revendication 2, dans lequel ladite solution comprenant AT-III est préparée par un procédé comprenant les étapes consistant à :
(i) préparer un surnageant I d'une fraction de Cohn à partir de plasma humain ;
(ii) mettre en contact ledit surnageant I de la fraction de Cohn avec un gel d'affinité capable de lier AT-III ; et
(iii) éluer et recueillir la fraction protéinique liée à ladite matrice d'affinité.

11. Procédé selon la revendication 10, dans lequel ledit gel d'affinité comprend l'héparine en tant que ligand d'affinité.

12. Procédé selon la revendication 1, dans lequel l'isoforme obtenue de AT-III est exempt de glycoprotéine riche en histidine (HRGP).
